# EUROPÄISCHE PATENTANMELDUNG

(11) **EP 2 335 671 A2**
(43) Veröffentlichungstag der Anmeldung: **22.06.2011**
(21) Anmeldenummer: 10191652.6
(22) Anmeldetag: 18.11.2010
(51) Int. Cl.: A61K 8/04, A61K 8/41, A61K 8/42, A61K 8/81, A61Q 5/00

(54) **Einphasige Haarkur**

(30) Priorität: 19.11.2009 DE 102009046873
(71) Anmelder: Henkel AG & Co. KGaA, 40589 Düsseldorf (DE)
(72) Erfinder: Delowsky, Jens, 22844, Norderstedt (DE); Battermann, Marlene, 21271, Asendorf (DE); Westphal, Petra, 21629, Neu Wulmstorf (DE); Hippe, Thomas, 25482, Appen (DE)

(57) **Zusammenfassung**

Es werden einphasige Haarkonditioniermittel, enthaltend ein kationisches Copolymer und ein Amidoamin und/oder ein kationisches Tensid, sowie die Verwendung dieser Mittel zur Haarpflege beschrieben.

## Beschreibung

Die Erfindung liegt auf dem Gebiet der kosmetischen Mittel und betrifft einphasige haarkonditionierende Mittel mit einem Gehalt an kationischen Copolymeren, kationischen Tensiden und/oder Aminen in einem kosmetisch akzeptablen Träger. Die Erfindung betrifft weiterhin die Verwendung des Mittels zur Haarpflege sowie ein Verfahren zur Haarpflege unter Verwendung des Mittels.

Nicht zuletzt durch die starke Beanspruchung der Haare, beispielsweise durch das Färben oder Dauerwellen, die Reinigung mit Shampoos und durch Umweltbelastungen, nimmt die Bedeutung von Pflegeprodukten mit möglichst lang anhaltender Wirkung zu.

Der Grund dafür ist, dass die Haare durch die Behandlung/Beanspruchung sowohl äußerlich, als auch in Ihrer Struktur geschädigt werden können, was ihnen ein unattraktives Aussehen verleiht, das sich durch mangelnde Glätte und Weichheit, mangelnden Glanz, aber auch durch eine schlechtere Kämmbarkeit, Haarbruch oder Spliss bemerkbar machen kann.

Daher ist es daher seit langem üblich, die Haare einer speziellen Nachbehandlung zu unterziehen, um den Haaren und der Kopfhaut Pflegestoffe zuzuführen, die den Haaren wieder ein ansprechendes Aussehen verleihen und die Haarstruktur kräftigen, sowie die Kopfhaut pflegen bzw. vor dem Austrocknen schützen. Bei solchen Nachbehandlungen werden, üblicherweise in Form einer Spülung, die Haare mit speziellen Wirkstoffen, beispielsweise quaternären Ammoniumsalzen oder speziellen Polymeren, behandelt. Durch diese Behandlung können je nach Formulierung die Kämmbarkeit, der Halt, die Fülle sowie der Glanz der Haare verbessert werden. Die Einarbeitung von Haut- und Haarkonditioniermitteln in eine kosmetisch geeignete - meist wässrige - Basis stellt die Hersteller solcher Zusammensetzungen aber immer wieder vor große Schwierigkeiten, denn insbesondere mineralische, natürliche oder synthetische Fett-, Wachs- und Ölkomponenten, die nachweislich einen pflegenden und konditionierenden Effekt auf der Haut und den Haaren hinterlassen, lassen sich nicht durch einfaches Einmischen in eine kosmetische Basis einarbeiten.

Insbesondere wenn Silikonöle und/oder pflanzliche Fette oder Öle in haarkosmetische Zusammensetzungen als Wirkstoffe eingearbeitet werden, erhält man üblicherweise mindestens zwei - oder mehrphasige Formulierungen. Die Bildung mehrphasiger Formulierungen ist umso wahrscheinlicher, je höher der Gehalt an Silikonölen und/oder pflanzlichen Fetten und Ölen in den Zusammensetzungen ist.

Die zwei- oder mehrphasigen Systeme müssen vor ihrer Anwendung von den Verbrauchern durch kräftiges Schütteln in ein nur für kurze Zeit stabiles einphasiges System überführt werden.

Selbst bei sachgemäßer Anwendung der Produkte ist dann eine gute Produktleistung auf dem Haar nicht immer gegeben, denn beispielsweise durch zu kurzes Schütteln kann nicht immer eine gleichmäßige Verteilung der Wirkstoffe gewährleistet werden was zur Folge haben kann, dass anstatt der erforderlichen Menge der Wirkstoffe mehr "Trägersubstanz" auf die Haare gelangt, wodurch die konditionierende Wirkung des Mittels sinkt.

Gleichzeitig kann bei einer solchen unsachgemäßen Anwendung das Haarkonditioniermittel nach jeder Anwendung konzentrierter werden, wodurch es ab einer gewissen Füllmenge sogar zu einer Überkonditionierung kommen kann, die zu schweren Haaren führt.

Es besteht daher der Bedarf nach stabilen einphasigen Haarkonditioniermitteln, die die Haare pflegen und deren Kämmbarkeit und Glanz erhöhen.

Es besteht weiterhin der Bedarf nach stabilen einphasigen Haarkonditioniermitteln, in die die Einarbeitung höherer Mengen an Ölkomponenten - insbesondere Silikonen und/oder pflanzlichen Ölen möglich ist, ohne dass die Einphasigkeit oder die gewünschte Viskosität der Mittel beeinträchtigt wird.

Insbesondere besteht der Bedarf nach solchen stabilen einphasigen Haarkonditioniermitteln, die sich für die Applikation aus einem Sprühapplikator eignen.

Vorgeschlagen werden Haarkonditioniermittel, die bestimmte kationische Polymere in der Kombination mit bestimmten Aminen und/oder kationischen Tensiden enthalten.

Die Haarkonditioniermittel sind einphasig und weisen eine geeignete Viskosität für eine Sprühapplikation auf.

Gegenstand der vorliegenden Erfindung ist daher ein einphasiges haarkonditionierendes Mittel, enthaltend
a) 0,1 bis 10 Gew.-% eines kationischen Copolymers, enthaltend Monomere der Formel (1) und Monomere der Formel (II) 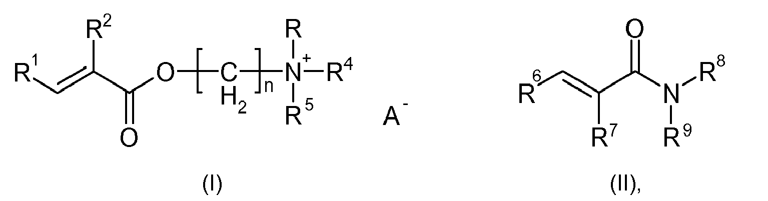 in welchen R¹ bis R⁹ unabhängig voneinander Wasserstoff, C₁-₄-Alkyl wie Methyl, Ethyl, Propyl, iso-Propyl, Butyl, iso-Butyl oder tert-Butyl bedeuten mit der Maßgabe, dass mindestens einer der Reste R⁶, R⁷, R⁸ oder R⁹ C₁-₄-Alkyl bedeutet, n für ganze Zahlen von 1 bis 8, und A⁻ für ein physiologisch verträgliches Anion wie Fluorid, Chlorid, Bromid, Iodid, Hydrogensulfat oder Methosulfat, steht,
b) 0,1 bis 10 Gew.-% mindestens einer Verbindung der Formel (lll), 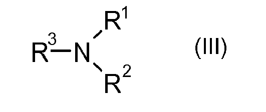 in der die Reste R¹ und R² unabhängig voneinander für Wasserstoff oder eine Alkylgruppe mit 1 bis 4 C-Atomen stehen, der Rest R³ für die Gruppe R⁴-CO(NH)-(CH₂)ₙ- steht, worin R⁴ eine gesättigte oder ungesättigte, verzweigte oder unverzweigte Alkylkette mit 7 bis 21 C-Atomen, und n eine ganze Zahl von 1 bis 4 bedeutet, und/oder
   0,01 bis 2 Gew.-% mindestes einer Verbindung der Formel (lV) 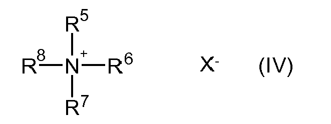 in der höchstens drei der Reste R⁵ bis R⁸ unanhängig voneinander für eine Alkylgruppe mit 1 bis 4 C-Atomen stehen, mindestens ein Rest R⁵ bis R⁸ für eine gesättigte oder ungesättigte, verzweigte oder unverzweigte Alkylkette mit 8 bis 30 C-Atomen steht, und worin X⁻ ein Halogenid oder eine Methosulfatgruppe bedeutet, und
c) einen kosmetischen Träger.

Die Inhaltsstoffe a), b) und c) werden nachfolgend detailliert beschrieben. Soweit nachstehend vom Wirkstoffkomplex (A) gesprochen wird, bezieht sich diese Aussage auf die in den erfindungsgemäßen Mitteln zwingend enthaltenen Inhaltsstoffe a), b) und c).

Ein wichtiger Aspekt des erfindungsgemäßen Haarkonditioniermittels ist, dass es versprühbar ist. Insbesondere soll sich das erfindungsgemäße Haarkonditioniermittel für eine Applikation aus einem Pump- und/oder Sprühspender eignen, so dass es durch einfache Betätigung eines Pump- oder Sprühventils auf die Haare gesprüht werden kann. Der große Vorteil einer solchen Applikationsform liegt darin, dass das Haarkonditioniermittel aus dem Sprühventil als feiner Sprühnebel heraustritt, der sich in jedem Bereich des Haares niederschlägt.

Damit das erfindungsgemäße Haarbehandlungsmittel versprühbar ist, und sich für die vorgenannte Applikationsform eignet, ist es von Vorteil, wenn es eine Viskosität von maximal 3000 mPas, bevorzugt von maximal 2750 mPas und insbesondere von maximal 2500 mPas aufweist (gemessen mit einem Brookfield-Viskosimeter DV-ll, Spindel 4 bei 20 UpM (20 s) und bei 20°C).

Bevorzugte erfindungsgemäße Haarkonditioniermittel sind frei von Treibmitteln.

Das erfindungsgemäße Haarkonditioniermittel wird - bevorzugt nach der Haarreinigung - fakultativ auf nasse oder trockene Haare gesprüht.

Ein geeigneter kosmetischer Träger c) für die erfindungsgemäßen Haarkonditioniermittel ist bevorzugt wässrig und/oder wässrig/alkoholisch. Bevorzugt enthält er mindestens 50 Gew.-% Wasser. Unter wässrig-alkoholischen kosmetischen Trägern sind wässrige Lösungen enthaltend 3 bis 70 Gew.-% eines C₁-C₆-Alkohols, insbesondere Methanol, Ethanol bzw. Propanol, Isopropanol, Butanol, Isobutanol, tert.-Butanol, n-Pentanol, iso-Pentanole, n-Hexanol, iso-Hexanol, Glykol, Glycerin, 1,2-Pentandiol, 1,5-Pentandiol, 1,2-Hexandiol oder 1,6-Hexandiol zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten.

In einer besonders bevorzugten Ausführungsform der Erfindung enthalten die Haarkonditioniermittel - bezogen auf ihr Gesamtgewicht - mindestens 70 Gew.-%, bevorzugt mindestens 75 Gew.-%, mehr bevorzugt mindestens 80 Gew.-% und insbesondere mindestens 85 Gew.-% eines wässrigen oder wässrig-alkoholischen Trägers.

Als Inhaltstoff a) enthalten die erfindungsgemäßen Mittel mindestens ein kationisches Copolymer. Das kationische Copolymer ist hierbei aus den Monomeren der Formel (I) und (II) aufgebaut, 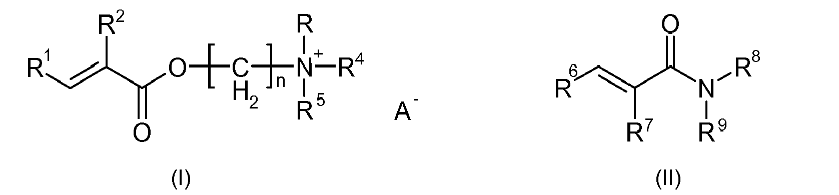 in welchen R¹ bis R⁹ unabhängig voneinander Wasserstoff, C₁-C₄-Alkyl wie Methyl-, Ethyl-, Propyl-, iso-Propyl-, Butyl-, iso-Butyl- oder tert-Butyl- bedeuten und der Maßgabe, dass mindestens einer der Reste R⁶, R⁷, R⁸ oder R⁹ C₁-C₄-Alkyl bedeutet, n ganzzahlig ist und für Zahlen von 1 bis 8 steht, und A⁻ für ein physiologisch verträgliches Anion wie Fluorid, Chlorid, Bromid, Iodid, Hydrogensulfat oder Methosulfat steht.

Vorzugsweise ist R¹ Wasserstoff oder eine Methylgruppe, besonders bevorzugt ist R¹ eine Methylgruppe. Vorzugsweise ist R² Wasserstoff oder eine C₁-C₄-Alkylgruppe, besonders bevorzugt ist R² Wasserstoff. Vorzugsweise sind R³, R⁴ und R⁵ unabhängig voneinander Wasserstoff oder eine C₁-C₄-Alkylgruppe, insbesondere eine Methylgruppe. Besonders bevorzugt sind R³, R⁴ und R⁵ gleich und höchst bevorzugt Methyl. Die Zahl n ist bevorzugt ganzzahlig und steht für Zahlen von 1 bis 7, bevorzugter von 1 bis 5, höchst bevorzugt 1, 2, 3 oder 4 und insbesondere für 2. R⁶ ist bevorzugt Wasserstoff oder Methyl. R⁷ ist bevorzugt Wasserstoff, Methyl oder Ethyl, besonders bevorzugt Methyl. R⁸ und R⁹ sind bevorzugt gleich und stehen für Wasserstoff, Methyl oder C₁-C₄-Alkyl, besonders bevorzugt Wasserstoff oder Methyl. Am bevorzugtesten steht das Monomer der Formel (II) für Dimethylacrylamid.

Das kationische Copolymer a) enthält bevorzugt 20 bis 95 Gew.-% Monomere der Formel (I) und 5 bis 50 Gew.% Monomere der Formel (ll).

Besonders bevorzugte kationische Copolymere a) enthalten 40 bis 90 Gew.-% Monomer (I) und 10 bis 40 Gew.-% Monomer (ll).

Das kationische Copolymer a) kann weiterhin vernetzt sein. In diesem Falle wird die Vernetzung durch die üblichen Vernetzungsmittel wie beispielsweise Allylacrylamid, Allylmethacrylamid, Tetraallylammoniumchlorid oder N,N'-methylen-bisacrylamid in Mengen bis zu 500 ppm bewirkt. Das kationische Copolymer a) kann weiterhin als eine Lösung, Suspension oder Dispersion in geeigneten kosmetischen Medien, wie beispielsweise Propylenglykol, Glycerin, Paraffin, Isoparaffin, Propylenglykolen und Estern von Propylenglykol wie beispielsweise Propylenglykol Dicaprylate/Dicaprate, oder PPG-1 Trideceth-6 und weiteren vorliegen.

Ein Beispiel für ein besonders bevorzugtes Copolymer a) ist das Handelsprodukt Tinovis^{®} CD der Firma Ciba.

Das kationische Copolymer a) wird in den erfindungsgemäßen Haarkonditioniermitteln bevorzugt in Mengen von 0,1 bis 5 Gew.-% und besonders bevorzugt in Mengen von 0,1 bis 3,0 Gew.-% eingesetzt - bezogen auf das Gewicht des Haarkonditioniermittels.

Bevorzugte erfindungsgemäße einphasige Haarkonditioniermittel sind solche, die bezogen auf ihr Gewicht -
a) 0,1 bis 5 Gew.-% des kationischen Copolymeren a), enthaltend Monomere der Formel (I) und Monomere der Formel (II),
b) 0,25 bis 5 Gew.-% einer Verbindung nach Formel (lll), worin die Reste R¹ und R² für Methylgruppen und der Rest R³ für die Gruppe R⁴-CO(NH)-(CH₂)ₙ- steht, in der R⁴ eine gesättigte oder ungesättigte, verzweigte oder unverzweigte Alkylkette mit 11 bis 19 C-Atomen, und n eine ganze Zahl von 2 oder 3 bedeutet, und/oder 0,05 bis 1 Gew.-% einer Verbindung nach Formel (lV), worin drei der Reste R⁵ bis R⁸ für eine Methylgruppe stehen, ein Rest R⁵ bis R⁸ für eine gesättigte oder ungesättigte, verzweigte oder unverzweigte Alkylkette mit 14 bis 26 C-Atomen steht und worin X⁻ Chlorid, Bromid oder eine Methosulfatgruppe bedeutet, und
c) mindestens 70 Gew.-%, bevorzugt mindestens 75 Gew.-%, mehr bevorzugt mindestens 80 Gew.-% und insbesondere mindestens 85 Gew.-% eines wässrigen oder wässrig-alkoholischen Trägers, enthalten.

Besonders bevorzugte Komponenten b) sind zum einen solche, die in der Formel (III) als Reste R¹ und R² Methylgruppen, als Rest R³ eine Gruppe R⁴-CO(NH)-(CH₂)ₙ-, als Rest R⁴ eine gesättigte oder ungesättigte, verzweigte oder unverzweigte Alkylkette mit 11 bis 19 C-Atomen enthalten, und in denen n die Zahl 3 bedeutet. Solche Verbindungen sind bekannt unter der Bezeichnung "Amidoamin".

Die Amidoamine können sowohl als solche in den Haarkonditioniermitteln vorliegen, als auch (infolge einer Protonierung in entsprechend saurer Lösung) als ihre entsprechende quaternäre Verbindung.

Erfindungsgemäß bevorzugt sind die nicht-kationischen Amidoamine.

Geeignete Amidoamine, welche gegebenenfalls quaternisiert sein können, sind: Tego Amid^{®} S18 (Evonik; INCI-Bezeichnung: Stearamidopropyl Dimethylamine), Lexamine^{®} S 13 (Inolex; INCI-Bezeichnung: Stearamidopropyl Dimethylamine), Incromine^{®} SB (Croda; INCI-Bezeichnung: Stearamidopropyl Dimethylamine), Witcamine^{®} 100 (Witco, INCI-Bezeichnung: Cocamidopropyl Dimethylamine), Incromine^{®} BB (Croda, INCI-Bezeichnung: Behenamidopropyl Dimethylamine), Mackine^{®} 401 (McIntyre, INCI-Bezeichnung: Isostearylamidopropyl Dimethylamine) und andere Mackine-Typen, Adogen^{Ⓡ} S18V (Witco, INCI-Bezeichnung: Stearylamidopropyl Dimethylamine). Als permanent kationische kommen in Frage: Aminoamine: Rewoquat^{®} RTM 50 (Witco Surfactants GmbH, INCI-Bezeichnung: Ricinoleamidopropyltrimonium Methosulfate), Empigen^{®} CSC (Albright&Wilson, INCI-Bezeichnung: Cocamidopropyltrimonium Chlorid), Swanol Lanoquat^{®} DES-50 (Nikko, INCI-Bezeichnung: Quatemium-33), Rewoquat^{®} UTM 50 (Witco Surfactants GmbH, Undecyleneamidopropyltrimonium Methosulfate).

Insbesondere bevorzugt ist Stearamidopropyl Dimethylamine.

Alternativ oder zusätzlich zu den vorgenannten Verbindungen der Formel (III) kann der Wirkstoff b) auch für ein kationisches Tensid stehen.

In diesem Fall ist als besonders bevorzugte Komponente b) eine Verbindung der Formel (lV) in den Haarkonditioniermitteln enthalten, in der drei Reste R⁵ bis R⁸ Methylgruppen sind und ein Rest R⁵ bis R⁸ einen unverzweigten Alkylrest mit einer Kettenlänge von 16 bis 22 darstellt. Bevorzugte quaternäre Ammoniumverbindungen der Formel (IV) sind Ammoniumhalogenide, insbesondere Chloride und Bromide, wie Alkyltrimethylammoniumchloride, Dialkyldimethylammoniumchloride und Trialkylmethylammoniumchloride, z.B. Distearyldimethylammoniumchlorid, Lauryltrimethylammoniumchlorid, Lauryltrimethylbenzylammoniumchlorid, Tricetylmethylammoniumchlorid Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat. Bevorzugt sind Cetyltrimethylammonium- und Behenyltrimethylammoniumsalze.

Besonders bevorzugt sind Cetyltrimethylammoniumsalze und insbesondere Cetyltrimethylammoniumchlorid und/oder Cetyltrimethylammoniummethosulfat.

In einer weiteren bevorzugten Ausführungsform der Erfindung können die erfindungsgemäßen Haarkonditioniermittel zur weiteren Steigerung der Haarpflege zusätzlich 0,05 bis 15 Gew.-%, bevorzugt 0,1 bis 12 Gew.-% und insbesondere 0,25 bis 10 Gew.-% mindestens einer Öl-, Wachs- und/oder Fettkomponente d) enthalten. Diese kann die bessere Kämmbarkeit der Haare unterstützen und als Rückfettungsmittel dienen.

Geeignete Öl-, Wachs- und/oder Fettkomponenten d) sind bevorzugt ausgewählt aus natürlichen und synthetischen Ölkomponenten und/oder Fettstoffen.

Als natürliche (pflanzliche) Öle werden üblicherweise Triglyceride und Mischungen von Triglyceriden eingesetzt. Bevorzugte natürliche Öle im Sinne der Erfindung sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Kakaoabutter und Shea-Butter. Insbesondere bevorzugt sind Mandelöl, Aprikosenkernöl, Arganöl, Olivenöl, Jojobaöl, Kakaobutter und Shea-Butter. Die pflanzlichen Öle können in den erfindungsgemäßen Haarkonditioniermitteln sowohl einzeln, als auch als Mischung mehrerer Öle eingesetzt werden.

Als mineralische Öle kommen insbesondere Mineralöle, Paraffin- und Isoparaffinöle sowie synthetische Kohlenwasserstoffe zum Einsatz. Ein geeigneter Kohlenwasserstoff ist beispielsweise das als Handelsprodukt erhältliche 1,3-Di-(2-ethylhexyl)-cyclohexan (Cetiol^{®} S).

Als synthetische Öle kommen weiterhin Silikonverbindungen in Betracht.

Silikone können auf dem Haar ausgezeichnete konditionierende Eigenschaften bewirken. Insbesondere können sie die Kämmbarkeit der Haare in nassem und trockenem Zustand verbessern und sich positiv auf den Haargriff und die Weichheit der Haare auswirken. Geeignete Silikone können ausgewählt sein aus der Gruppe der:
(i) Polyalkylsiloxane, Polyarylsiloxane, Polyalkylarylsiloxane, die flüchtig oder nicht flüchtig, geradkettig, verzweigt oder cyclisch, vernetzt oder nicht vernetzt sind;
(ii) Polysiloxane, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmere vom Typ (A-B)ₙ mit n > 3;
(iv) gepfropften Siliconpolymere mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymere mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder der Gemische dieser Gruppen.

Als Ölkomponente kann weiterhin ein Dialkylether dienen.

Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Besonders bevorzugt ist der Di-n-octylether, der im Handel unter der Bezeichnung Cetiol^{®} OE erhältlich ist.

In einer weiteren bevorzugten Ausführungsform kann die Wirkung der Wirkstoffkombination A durch weitere Fettstoffe noch weiter optimiert werden. Unter weiteren Fettstoffen sind Fettsäuren, Fettalkohole sowie natürliche und synthetische Wachse zu verstehen, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist Stearinsäure.

Als Fettalkohole können gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂-Kohlenstoffatomen eingesetzt werden. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb**^{®}** 12, Isocarb^{®} 16 oder Isocarb^{®} 24 käuflich zu erwerben. Selbstverständlich können auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Als natürliche oder synthetische Wachse können feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP eingesetzt werden. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD.

Besonders bevorzugt im Hinblick auf den Haarglanz und eine verbesserte Kämmbarkeit sind erfindungsgemäße Haarkonditioniermittel, die als weitere haarkonditionierende Komponente d) mindestens ein Silikonöl - vorzugsweise ein Dimethicon - und/oder mindestens ein pflanzliches Öl enthalten. Diese Öle lassen sich in die erfindungsgemäßen Haarkonditioniermittel einzeln oder als Gemisch in einer Menge bis zu 15 Gew.-% einarbeiten, ohne dass die Einphasigkeit und/oder die Stabilität der Mittel darunter leiden. Des Weiteren wird die erforderliche Viskosität der Mittel für eine Sprühapplikation trotz des hohen Gehalts der vorgenannten bevorzugten haarkonditionierenden Öle erreicht.

Vitamine sind für die Gesundheit von Haut und Haaren unentbehrlich. Da sie auch bei äußerer Anwendung eine günstige Wirkung zeigen, werden sie kosmetischen Präparaten oftmals zugesetzt. Es wurde gefunden, dass das kationische Copolymer a) in den Haarkonditioniermitteln neben der Abscheidung der Komponenten b) und d) auf den Haaren auch die Abscheidung weiterer Pflegestoffe - wie auch Vitaminen - stimulieren kann.

Bevorzugte Vitamine, Provitamine und Vitaminvorstufen sowie deren Derivate, die in den erfindungsgemäßen Haarkonditioniermitteln eingesetzt werden können, sind den Gruppen A, B, C, E, F und H zuzuordnen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B_{3.} Unter dieser Bezeichnung werden häufig die Verbindungen Nicotinsäure und Nicotinsäureamid (Niacinamid) geführt. Bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Für das Vitamin H hat sich der Trivialname Biotin durchgesetzt. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt und können den erfindungsgemäßen Haarkonditioniermitteln sowohl einzeln, als auch in ihrer Kombination zugesetzt werden.

Besonders bevorzugt sind erfindungsgemäße Haarkonditioniermittel, die, bezogen auf ihr Gewicht,
a) 0,25 bis 3 Gew.-% Tinovis^{®}CD,
b) 0,25 bis 3 Gew.-% Stearamidopropyl Dimethylamine und/oder
   0,05 bis 1 Gew.-% Cetyltrimethylammoniumchlorid,
c) 0,5 bis 10 Gew.-% Dimethicone und/oder pflanzliches Öl und
d) 0,05 bis 0,5 Gew.-% eines oder mehrerer Vitamine/Vitaminvorstufen aus der Gruppe

Panthenol, Pantolacton, Nicotinsäureamid und/oder Biotin enthalten.

Ein weiterer fakultativer Wirkstoff in den erfindungsgemäßen Zusammensetzungen ist mindestens ein Proteinhydrolysat und/oder dessen Derivate. Proteinhydrolysate können die Kräftigung der Haarstruktur unterstützen und das Austrocknen der Haare verhindern.

Geeignete Proteinhydrolysate können sowohl pflanzlichen als auch tierischen, marinen oder synthetischen Ursprungs sein.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein⁵ (Inolex) und Kerasol^{®} (Croda) vertrieben.

Bevorzugte pflanzliche Proteinhydrolysaten sind beispielsweise Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Weitere bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate. Beispiele für erfindungsgemäße Perlenhydrolysate sind die Handelsprodukte Pearl Protein Extract BG^{®} oder Crodarom^{®} Pearl.

Besonders bevorzugt werden in den erfindungsgemäßen Haarkonditioniermitteln Keratin-, Seiden-, Milcheiweiß-, Weizen- und/oder Sojaproteinhydrolysate eingesetzt. Insbesondere bevorzugt sind Keratin- und/oder Weizenproteinhydrolysate.

Die Proteinhydrolysate sind in den Zusammensetzungen - bezogen auf deren Gesamtgewicht - vorzugsweise in Mengen von 0,05 Gew.-% bis zu 15 Gew.-% und insbesondere in Mengen von 0,05 Gew.-% bis zu 5 Gew.-% enthalten.

Weiterhin besonders bevorzugt sind erfindungsgemäße Haarkonditioniermittel, die - bezogen auf ihr Gewicht -
a) 0,25 bis 3 Gew.-% Tinovis^{®}CD,
b) 0,25 bis 3 Gew.-% Stearamidopropyl Dimethylamine und/oder
   0,05 bis 1 Gew.-% Cetyltrimethylammoniumchlorid,
c) 0,5 bis 10 Gew.-% Dimethicone und/oder pflanzliches Öl,
a) 0,05 bis 0,5 Gew.-% eines oder mehrerer Vitamine/Vitaminvorstufen aus der Gruppe Panthenol, Pantolacton, Nicotinsäureamid und/oder Biotin und
b) 0,05 bis 5 Gew.-% eines oder mehrerer Proteinhydrolysate aus der Gruppe Keratin-, Seiden-, Milcheiweiß-, Weizen- und/oder Sojaproteinhydrolysate
enthalten.

Eine weitere fakultative Komponente zur Stabilisierung der erfindungsgemäßen Haarkonditioniermittel ist ein nichtionisches Tensid und/oder ein nichtionischer Emulgator. Nichtionische Tenside und/oder nichtionische Emulgatoren werden den erfindungsgemäßen Haarkonditioniermitteln - bezogen auf deren Gewicht - einzeln oder in ihrer Kombination bevorzugt in Mengen von 0,05 bis 7,5 Gew.-% und insbesondere in Mengen von 0,1 bis 5 Gew.-% zugegeben.

Geeignete nichtionische Tenside enthalten als hydrophile Gruppe z. B. eine Polyolgruppe, eine Polyalkylenglykolethergruppe oder eine Kombination aus einer Polyol- und einer Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂-C₆- Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl, beispielsweise Rizinusöl-hydriert+40 EO, wie es beispielsweise unter dem Handelsnamen Cremophor CO 455 von der Firma SHC im Handel erhältlich ist,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (V)

   R¹⁴CO―(OCH₂CHR¹⁵)_{w}OR¹⁶ (V)

   in der R¹⁴CO für einen linearen oder verzweigten, gesättigten und/oder ungesättigten Acylrest mit 6 bis 22 Kohlenstoffatomen, R¹⁵ für Wasserstoff oder Methyl, R¹⁶ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside. Alkylpolyglucoside entsprechen der allgemeinen Formel RO-(Z)ₓ wobei R für Alkyl, Z für Zucker sowie x für die Anzahl der Zuckereinheiten steht. Die in den erfindungsgemäßen Mitteln einsetzbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Besonders geeignete Alkylpolyglykoside enthalten im Schnitt 1,1 bis 5 Zucker einheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Besonders bevorzugte nichtionische Tenside sind Alkylpolyglucoside und Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure. Zubereitungen mit hervorragenden milden Eigenschaften können ebenfalls erhalten werden, wenn sie als nichtionische Tenside Fettsäureester von ethoxyliertem Glycerin enthalten.

Erfindungsgemäß geeignete Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Weiterhin können die erfindungsgemäßen Haarkonditioniermittel zusätzlich zu der zwingenden Komponente b) mindestens ein weiteres kationisches Tensid enthalten.

Geeignete weitere - von b) verschiedene - kationische Tenside sind beispielsweise die sogenannten Esterquats, wie beispielsweise die unter den Warenzeichen Rewoquat^{®}, Stepantex^{®}, Dehyquart^{®} und Armocare^{®} vertrieben Produkte. Die Produkte Armocare^{®} VGH-70, ein N,N-Bis(2-Palmitoyloxyethyl)dimethylammonium-chlorid, sowie Dehyquart^{®} F-75, Dehyquart^{®} C-4046, Dehyquart^{®} L80, Dehyquart^{®} F-30, Dehyquart^{®} AU-35, Rewoquat^{®} WE18, Rewoquat^{®} WE38 DPG und Stepantex^{®} GS 90 sind Beispiele für besonders bevorzugte Esterquats.

Weitere bevorzugte kationische Tenside zählen zu den kationischen Betainestern, wie beispielsweise die Handelsprodukte sind Schercoquat^{®} BAS, Lexquat^{®} AMG-BEO, Akypoquat^{®} 131 oder Incroquat^{®} Behenyl HE.

Schließlich können die weiteren kationischen Tenside quartäre Imidazolinverbindungen sein, d.h. Verbindungen, die einen positiv geladenen Imidazolinring aufweisen.

Beispiele für besonders bevorzugte quartäre Imidazolinverbindungen sind unter den INCI - Bezeichnungen Quaternium-27, Quaternium-72, Quaternium-83 und Quaternium-91 bekannt.

Selbstverständlich umfasst die erfindungsgemäße Lehre auch, dass mindestens zwei weitere kationische Tenside verwendet werden können. In diesem Falle ist es bevorzugt, wenn die kationischen Tenside aus zwei unterschiedlichen Strukturklassen gewählt werden.

Die weiteren - von b) verschiedenen - kationischen Tenside können in den erfindungsgemäßen Zusammensetzungen bevorzugt in Mengen von 0,01 bis 10 Gew.-% und insbesondere in Mengen von 0,1 bis 7,5 Gew.-% enthalten sein. Die besten Ergebnisse werden dabei mit Mengen von 0,1 bis 5 Gew.-%, jeweils bezogen auf das Gewicht der Gesamtzusammensetzung, erhalten.

In den erfindungsgemäßen Mitteln können als weitere haarkonditionierende Wirkstoffe auch Polymere eingesetzt werden, die kationisch und/oder amphoter und/oder zwitterionisch sein können.

Im Folgenden werden einige Beispiele von besonders bevorzugten Polymeren beschrieben.

Geeignete weitere - von a) verschiedene - kationische Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat^{®} 755 N und Gafquat^{®} 734 von der Firma Gaf Co., USA vertrieben wird und von denen das Gafquat^{®} 734 besonders bevorzugt ist, geeignet. Weitere kationische Polymere sind beispielsweise das von der Firma BASF, Deutschland unter dem Handelsnamen Luviquat^{®} HM 550 vertriebene Copolymer aus Polyvinylpyrrolidon und Imidazoliminmethochlorid, das von der Firma Calgon/USA unter dem Handelsnamen Merquat^{®} Plus 3300 vertriebene Terpolymer aus Dimethyldiallylammoniumchlorid, Natriumacrylat und Acrylamid und das von der Firma ISP unter dem Handelsnamen Gafquat^{®} HS 100 vertriebene Vinylpyrrolidon/Methacrylamidopropyltrimethylammoniumchlorid Copolymer.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (3V Sigma) im Handel erhältlich. Weitere Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Ein bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate.

Eine kationische Cellulose wird unter der Bezeichnung Polymer JR^{®} 400 von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Weitere Handelsprodukte sind die Verbindungen Celquat^{®} H 100, Celquat^{®} und L 200. Ebenfalls erfindungsgemäß bevorzugt ist eine kationische Cellulose, die unter der INCI-Bezeichnung Polyquaternium-67 (SoftCat) bekannt ist.

Geeignete kationische Guarderivate werden unter der Handelsbezeichnung Jaguar^{®} vertrieben und haben die INCI-Bezeichnung Guar Hydroxypropyltrimonium Chloride. Weiterhin werden besonders geeignete kationische Guarderivate auch von der Fa. Hercules unter der Bezeichnung N-Hance^{®} im Handel. Weitere kationische Guarderivate werden von der Fa. Cognis unter der Bezeichnung Cosmedia^{®} vertrieben. Ein bevorzugtes kationisches Guarderivat ist das Handelsprodukt AquaCat^{®} der Fa. Hercules. Bei diesem Rohstoff handelt es sich um ein bereits vorgelöstes kationisches Guarderivat.

Ein geeignetes Chitosan wird beispielsweise von der Firma Kyowa Oil& Fat, Japan, unter dem Handelsnamen Flonac^{®} vertrieben. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Weitere Chitosanderivate sind unter den Handelsbezeichnungen Hydagen^{®} CMF, Hydagen^{®} HCMF und Chitolam^{®} NB/101 im Handel frei verfügbar.

Weitere bevorzugte kationische Polymere sind beispielsweise
- kationische Alkylpolyglycoside,
- kationisierter Honig, beispielsweise das Handelsprodukt Honeyquat^{®} 50,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium-2, Polyquaternium-17, Polyquaternium-18 und Polyquaternium-27 bekannten Polymeren mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Ebenfalls verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Besonders bevorzugte amphotere Polymere sind Copolymere, aus Diallyldimethylammoniumchlorid und Acrylsäure. Diese Copolymerisate werden unter der INCI-Bezeichnung Polyquaternium-22 unter anderem mit dem Handelsnamen Merquat^{®} 280 (Nalco) vertrieben. Ganz besonders bevorzugte amphotere Polymere sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat^{®} Plus 3330 (Nalco) vertrieben.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.

Die weiteren Polymere (P) können in den erfindungsgemäßen Zusammensetzungen bevorzugt in Mengen von 0,01 bis 30 Gew.-%, bezogen auf die gesamte Zusammensetzung, enthalten sein. Zusätzlich kann es sich als vorteilhaft erweisen, wenn in den erfindungsgemäßen Zusammensetzungen Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Eine besonders bevorzugte Gruppe von Quellmitteln können Hydantoine sein. Erfindungsgemäße Zusammensetzungen enthalten bevorzugt 0,01 bis 5 Gew.-% Hydantoin bzw. mindestens eines Hydatoinderivats. Besonders bevorzugt werden Hydantoinderivate eingesetzt, wobei 5-Ureidohydantoin besonders bevorzugt ist. Unabhängig davon, ob Hydantoin oder Hydantoinderivat(e) eingesetzt wird/werden, sind Einsatzmengen von 0,02 bis 2,5 Gew.-% bevorzugt, von 0,05 bis 1,5 Gew.-% besonders bevorzugt und von 0,075 bis 1 Gew.-% insbesondere bevorzugt.

Ferner können die kosmetischen Mittel weitere Wirk-, Hilfs- und Zusatzstoffe enthalten, wie beispielsweise
- Strukturanten wie Maleinsäure und Milchsäure,
- Parfümöle, Dimethylisosorbid und Cyclodextrine,
- Farbstoffe zum Anfärben des Mittels,
- Antischuppenwirkstoffe wie Piroctone Olamine, Zink Omadine und Climbazol,
- Cholesterin,
- Komplexbildner wie EDTA, NTA, β-Alanindiessigsäure und Phosphonsäuren,
- Pigmente,
- Antioxidantien.

Die erfindungsgemäßen einphasigen Haarkonditioniermittel weisen vorzugsweise einen pH-Wert im aciden Bereich von 2 bis 6,5, bevorzugt von 2,4 bis 6,0 und insbesondere von 2,8 bis 5,5 auf. Die erfindungsgemäßen Mittel eignen sich insbesondere für die Haarpflege und verbessern die Kämmbarkeit, die Elastizität, das Volumen und den Glanz der Haare.

Diese positiven Eigenschaften werden sowohl bei einer typischen ausspülbaren (rinse-off) als auch bei einer auf dem Haar verbleibenden (leave-on) Applikation erhalten.

In hervorragender Weise eignet sich die erfindungsgemäße Zusammensetzung jedoch für eine leave-on-Sprühapplikation. Hierbei wird gegenüber den bislang üblichen zweiphasigen Produkten ein außerordentlich gleichmäßiges Sprühbild erzielt. Dadurch wird die gesamte Zusammensetzung sehr gleichmäßig auf dem Haar verteilt.

Ein zweiter Gegenstand der Erfindung ist die kosmetische Verwendung des erfindungsgemäßen einphasigen Haarkonditioniermittels zur Erhöhung der Haarelastizität, des Haarglanzes und des Haarvolumens sowie zur Verbesserung der Kämmbarkeit.

Ein dritter Gegenstand der Erfindung ist ein kosmetisches Verfahren zur Pflege von Haaren, bei dem das erfindungsgemäße einphasige Haarkonditioniermittel - vorzugsweise nach der Haarreinigung - auf die trockenen oder nassen Haare gesprüht und bis zur nächsten Haarreinigung auf den Haaren belassen wird.

Bevorzugt liegt das erfindungsgemäße Haarkonditioniermittel dabei als Non-Aerosol-Sprühkur vor.

### Beispiel

### Non Aerosol Sprühconditioner

| | Beispiel 1 | Beispiel 2 | Beispiel 3 |
|---|---|---|---|
| Dimethicone | 5,00 | 10,00 | 0,50 |
| Tinovis^{®1}CD | 1,50 | 2,50 | 2,30 |
| Stearamidopropyldimethylamine | 1,00 | 0,50 | 1,50 |
| Gluadin^{®2} WQ | 1,50 | 1,50 | 1,50 |
| D-Panthenol, 75% | 0,20 | 0,20 | 0,20 |
| Glycerin | 0,10 | - | - |
| Dehyquart^{®3} A CA | - | 0,40 | - |
| Aprikosenkernöl | - | - | 4,00 |
| PEG-40 Hydrogenated Castor Oil | - | - | 0,60 |
| Nicotinsäureamid | - | - | 0,20 |
| Parfüm | 0,80 | 0,35 | 0,20 |
| Benzophenone-4 | 0,40 | - | - |
| Puffer | 0,50 | 0,50 | 0,50 |
| Konservierung | q.s. | q.s. | q.s. |
| Wasser, demineralisiert | ad 100 | ad 100 | ad 100 |

Die Haarkonditioniermittel der Beispiele 1 bis 3 wurden mittels eines handelsüblichen Pumpspenders auf die nassen oder trockenen Haare gesprüht.

Es wurden die folgenden Handelsprodukte eingesetzt:
- 1: INCI-Bezeichnung: Dimethylacrylamide/Ethyltrimonium Chloride Methacrylate Copolymer, Propylene Glycol Dicaprylate/Dicaprate, PPG-1 Trideceth-6, C10-11 Isoparaffin; BASF,
- 2: INCI-Bezeichnung: AQUA (WATER), LAURDIMONIUM HYDROXYPROPYL HYDROLYZED WHEAT PROTEIN, Cognis,
- 3: INCI-Bezeichnung: AQUA (WATER), CETRIMONIUM CHLORIDE, Cognis.

## Patentansprüche

1. Einphasiges haarkonditionierendes Mittel, enthaltend - bezogen auf sein Gewicht -
a) 0,1 bis 10 Gew.-% eines kationischen Copolymers, enthaltend Monomere der Formel (I) und Monomere der Formel (II) 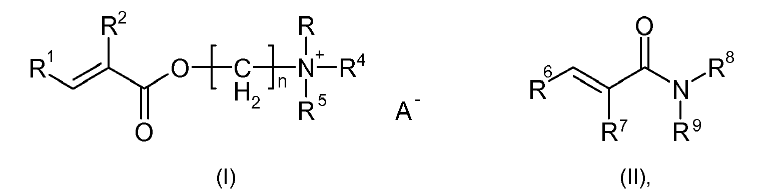 in welchen R¹ bis R⁹ unabhängig voneinander Wasserstoff, C₁-₄-Alkyl wie Methyl, Ethyl, Propyl, iso-Propyl, Butyl, iso-Butyl oder tert-Butyl bedeuten mit der Maßgabe, dass mindestens einer der Reste R⁶, R⁷, R⁸ oder R⁹ C₁-₄-Alkyl bedeutet, n für ganze Zahlen von 1 bis 8, und A⁻ für ein physiologisch verträgliches Anion wie Fluorid, Chlorid, Bromid, Iodid, Hydrogensulfat oder Methosulfat, steht,
b) 0,1 bis 10 Gew.-% mindestens einer Verbindung der Formel (lll), 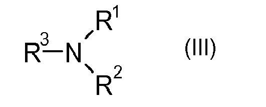 in der die Reste R¹ und R² unabhängig voneinander für Wasserstoff oder eine Alkylgruppe mit 1 bis 4 C-Atomen stehen, der Rest R³ für die Gruppe
R⁴-CO(NH)-(CH₂)ₙ- steht, worin R⁴ eine gesättigte oder ungesättigte, verzweigte oder unverzweigte Alkylkette mit 7 bis 21 C-Atomen, und n eine ganze Zahl von 1 bis 4 bedeutet, und/oder
0,01 bis 2 Gew.-% mindestes einer Verbindung der Formel (IV) 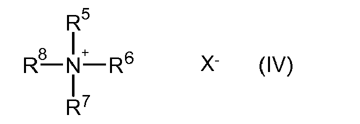 in der höchstens drei der Reste R⁵ bis R⁸ unanhängig voneinander für eine Alkylgruppe mit 1 bis 4 C-Atomen stehen, mindestens ein Rest R⁵ bis R⁸ für eine gesättigte oder ungesättigte, verzweigte oder unverzweigte Alkylkette mit 8 bis 30 C-Atomen steht, und worin X⁻ ein Halogenid oder eine Methosulfatgruppe bedeutet, und
c) einen kosmetischen Träger.

2. Haarkonditioniermittel nach Anspruch 1, **dadurch gekennzeichnet, dass** es eine Viskosität von maximal 3000 mPas, bevorzugt von maximal 2750 mPas und insbesondere von maximal 2500 mPas aufweist (gemessen mit einem Brookfield-Viskosimeter DV-ll, Spindel 4 bei 20 UpM (20 s) und bei 20°C) und versprühbar ist.

3. Haarkonditioniermittel nach einem der Ansprüche 1 oder 2, **dadurch gekennzeichnet, dass** es - bezogen auf sein Gewicht -
a) 0,1 bis 5 Gew.-% des kationischen Copolymeren a), enthaltend Monomere der Formel (I) und Monomere der Formel (II),
b) 0,25 bis 5 Gew.-% einer Verbindung nach Formel (lll), worin die Reste R¹ und R² für Methylgruppen und der Rest R³ für die Gruppe R⁴-CO(NH)-(CH₂)ₙ- steht, in der R⁴ eine gesättigte oder ungesättigte, verzweigte oder unverzweigte Alkylkette mit 11 bis 19 C-Atomen, und n eine ganze Zahl von 2 oder 3 bedeutet, und/oder 0,05 bis 1 Gew.-% einer Verbindung nach Formel (lV), worin drei der Reste R⁵ bis R⁸ für eine Methylgruppe stehen, ein Rest R⁵ bis R⁸ für eine gesättigte oder ungesättigte, verzweigte oder unverzweigte Alkylkette mit 14 bis 26 C-Atomen steht und worin X⁻ Chlorid, Bromid oder eine Methosulfatgruppe bedeutet, und
c) mindestens 70 Gew.-%, bevorzugt mindestens 75 Gew.-%, mehr bevorzugt mindestens 80 Gew.-% und insbesondere mindestens 85 Gew.-% eines wässrigen oder wässrig-alkoholischen Trägers, enthält.

4. Haarkonditioniermittel nach einem der Ansprüche 1 bis 3 **dadurch gekennzeichnet, dass** es - bezogen auf sein Gesamtgewicht - zusätzlich 0,05 bis 15 Gew.-%, bevorzugt 0,1 bis 12 Gew.-% und insbesondere 0,25 bis 10 Gew.-% mindestens einer Öl-, Wachs- und/oder Fettkomponente enthält.

5. Haarkonditioniermittel nach Anspruch 4, **dadurch gekennzeichnet, dass** es als Öl-, Wachs- und/oder Fettkomponente mindestens ein Silikon und/oder mindestens ein pflanzliches Öl enthält.

6. Haarkonditioniermittel nach einem der Ansprüche 1 bis 5, **dadurch gekennzeichnet, dass** es zusätzlich mindestens einen Stoff aus der Gruppe der Vitamine, Provitamine und Vitaminvorstufen und/oder mindestens einen Stoff aus der Gruppe der Proteinhydrolysate enthält.

7. Kosmetische Verwendung eines Haarkonditioniermittels nach einem der Ansprüche 1 bis 6 zur Erhöhung der Haarelastizität, des Haarglanzes, des Haarvolumens sowie zur Verbesserung der Kämmbarkeit.

8. Kosmetisches Verfahren zur Pflege von Haaren, **dadurch gekennzeichnet, dass** ein Haarkonditioniermittel nach einem der Ansprüche 1 bis 6 - vorzugsweise nach der Haarreinigung - auf die trockenen oder nassen Haare gesprüht und bis zur nächsten Haarreinigung auf den Haaren belassen wird.
